# EUROPÄISCHE PATENTSCHRIFT

(11) **EP 3 006 063 B1**
(45) Veröffentlichungstag und Bekanntmachung des Hinweises auf die Patenterteilung: **08.11.2017**
(21) Anmeldenummer: 15183552.7
(22) Anmeldetag: 02.09.2015
(51) Int. Cl.: A61M 5/162, A61J 1/14, A61J 1/20, A61M 39/04, A61M 39/26

(54) **VERSCHLUSSANORDNUNG FÜR EIN TRÄGERGEHÄUSE EINES MEDIZINISCHEN FLUIDSPEICHER- UND/ODER -LEITUNGSSYSTEMS**
CLOSURE ASSEMBLY FOR A CARRIER HOUSING OF A MEDICAL FLUID STORAGE AND/OR CONDUIT SYSTEM
DISPOSITIF DE FERMETURE POUR UN BOITIER DE SUPPORT D'UN SYSTEME DE CONDUITE ET/OU DE STOCKAGE DE FLUIDE MEDICAL

(30) Priorität: 15.09.2014 DE 102014218414
(43) Veröffentlichungstag der Anmeldung: 13.04.2016
(73) Patentinhaber: B. Braun Melsungen AG, 34212 Melsungen (DE)
(72) Erfinder: BONNAL, Olivier, 34212 Melsungen (DE)
(74) Vertreter: Patentanwälte Ruff, Wilhelm, Beier, Dauster & Partner mbB

(56) Entgegenhaltungen:
- WO-A1-94/03373
- WO-A1-97/39720
- WO-A1-2009/035383
- DE-A1-102005 015 504
- US-A- 3 987 791
- US-A- 5 232 109
- US-A- 5 895 383

## Beschreibung

Die Erfindung betrifft eine Verschlussanordnung für ein Trägergehäuse eines medizinischen Fluidspeicher- und/oder -leitungssystems mit einem Aufnahmestutzen, in dem eine elastisch verformbare Verschlussmembran angeordnet ist, die so gestaltet ist, dass sie einen eingesteckten Dorn eines Anschlussteils kraftschlüssig sichert, wobei die Verschlussmembran wenigstens ein mechanisches Rückhaltemittel aufweist, das auf den Dorn des Anschlussteils in eingestecktem Zustand derart wirkt, dass höhere Kräfte zum Herausziehen des Dorns aus der Verschlussmembran notwendig sind als die Kräfte, die für ein Hineinstecken des Dorns benötigt sind, und wobei das wenigstens eine Rückhaltemittel ein separates Rückhalteelement aus Kunststoff umfasst, das lösbar oder unlösbar mit der Verschlussmembran verbunden ist.

Eine derartige Verschlussanordnung ist aus der US 5,232,109 bekannt. Die bekannte Verschlussanordnung ist für ein Trägergehäuse eines medizinischen Fluidspeichersystems vorgesehen und weist eine elastisch verformbare Verschlussmembran auf. Die elastisch verformbare Verschlussmembran weist einen Einstechkanal auf, der im Bereich seiner Innenwandung profiliert ist.

Eine weitere Verschlussanordnung ist aus der US 5,520,641 allgemein bekannt. Die Verschlussanordnung ist Teil eines Trägergehäuses, das in einem medizinischen Infusionssystem integriert ist. Das Trägergehäuse weist einen Aufnahmestutzen auf, der durch eine stopfenartige, aus einem Elastomer bestehende Verschlussmembran verschlossen ist. Das Elastomer der Verschlussmembran kann durch einen Dorn oder eine Kanüle eines Fluidspeicher- oder - leitungselements durchdrungen werden, um einen Fluiddurchtritt durch den Dorn bzw. die Kanüle und damit durch die Verschlussmembran hindurch zu ermöglichen. Beim Hindurchstecken des Dorns oder der Kanüle durch die Verschlussmembran wird das Material der Verschlussmembran elastisch verformt, wobei der Dorn bzw. die Kanüle dicht von dem elastisch verformten Material der Verschlussmembran umschlossen ist. Die elastisch verformte Verschlussmembran sichert den Dorn oder die Kanüle kraftschlüssig an dem Aufnahmestutzen. Der Dorn bzw. die Kanüle kann durch Herausziehen von dem Aufnahmestutzen entfernt werden.

Aufgabe der Erfindung ist es, eine Verschlussanordnung der eingangs genannten Art zu schaffen, die eine verbesserte Handhabung durch medizinisches Pflegepersonal und eine größere Sicherheit für das medizinische Pflegepersonal ermöglicht.

Diese Aufgabe wird dadurch gelöst, dass das Rückhalteelement als elastisch verformbarer Hohlkörper gestaltet ist, der an seinem Innenumfang Rückhalteprofilierungen aufweist, deren Profilflanken in Einsteckrichtung steiler gestaltet sind als in Entnahmerichtung. Vorzugsweise sind die auf den Dorn in eingestecktem Zustand in der Verschlussmembran wirkenden Rückhaltekräfte wenigstens doppelt so hoch wie die Kräfte, die durch medizinisches Pflegepersonal zum Einstechen oder Einstecken des Dorns in die Verschlussmembran notwendig sind. Durch die erfindungsgemäße Lösung ist zum einen eine einfache Handhabung für das medizinische Pflegepersonal beim Einstechen des Dorns in die Verschlussmembran möglich. Denn das Einstechen erfolgt mit manuellen Kräften, die dem Pflegepersonal bei entsprechenden Einstechvorgängen in Verschlussanordnungen aus dem Stand der Technik bekannt sind. Durch das wenigstens eine Rückhaltemittel ist sichergestellt, dass sich der Dorn nicht mehr von dem Aufnahmestutzen lösen kann. Dies ist insbesondere vorteilhaft, falls der Dorn Teil eines Anschlusselements eines medizinischen Fluidleitungssystems ist, der in die Verschlussanordnung einer Flasche oder eines Beutels eingestochen wird, in denen eine Infusionslösung gespeichert ist. Denn durch die Erfindung wird ein unbeabsichtigtes Lösen des Dorns von der Verschlussanordnung vermieden, so dass auch kein Hautkontakt des Pflegepersonals mit dieser Infusionslösung auftreten kann. Durch die erfindungsgemäße Lösung ist der Dorn gegen ein Herausziehen gesichert, sobald er in die Verschlussmembran eingestochen ist. In vorteilhafter Weise ist das Trägergehäuse, in dem die erfindungsgemäße Verschlussanordnung vorgesehen ist, Teil eines medizinischen Fluidspeicherbehältnisses in Form einer Flasche oder eines Beutels. In montiertem Zustand ist ein derartiges Fluidspeicherbehältnis vorzugsweise mit der Verschlussanordnung nach unten ausgerichtet, um eine luftblasenfreie Entnahme des Fluids des Fluidspeicherbehältnisses zu ermöglichen. Die Verschlussmembran ist aus einem elastisch verformbaren Material, vorzugsweise aus einem Elastomer, insbesondere aus Silikon, oder aus einem geeigneten thermoplastischen Elastomer hergestellt. Erfindungsgemäß umfasst das wenigstens eine Rückhaltemittel ein separates Rückhalteelement aus Kunststoff, das lösbar oder unlösbar mit der Verschlussmembran verbunden ist. Dadurch, dass sowohl die Verschlussmembran als auch das Rückhalteelement aus Kunststoff hergestellt sind, weist die Verschlussanordnung keinerlei metallische Elemente oder Abschnitte auf, die die Qualität des im medizinischen Fluidsystem geführten und gespeicherten Fluids beeinträchtigen könnten.

In Ausgestaltung der Erfindung ist der Hohlkörper konisch gestaltet, und die Verschlussmembran weist eine komplementär konische Aussparung auf, in die der Hohlkörper eintaucht. Durch die konische Gestaltung ergibt sich ein unterschiedlich hoher Kraftschluss längs der Steckachse je nachdem, ob der Dorn in die Verschlussmembran und in den Hohlkörper eingesteckt oder in Entnahmerichtung gezogen wird.

In weiterer Ausgestaltung der Erfindung verjüngt die konische Form des Hohlkörpers und der Aussparung sich von der Innenseite der Verschlussmembran in Richtung zur Außenseite. Dadurch ist bei einer Steckbewegung des Dorns zur Innenseite der Verschlussmembran hin ein geringerer Kraftaufwand nötig als bei einer entgegengerichteten Herausziehbewegung des Dorns in Richtung zur Außenseite der Verschlussmembran.

In weiterer Ausgestaltung der Erfindung ist der Hohlkörper mit über seinen Umfang verteilt angeordneten seitlichen Längsschlitzen versehen, die eine elastische Deformation unterstützen. Die Längsschlitze erstrecken sich längs der Steckachse im Mantel des Hohlkörpers.

In weiterer Ausgestaltung der Erfindung ist die Aussparung zur Aufnahme des Hohlkörpers mit Längsprofilierungen versehen. In weiterer Ausgestaltung ist ein Außenmantel des Hohlkörpers mit komplementären Längsprofilierungen versehen, um eine Gleitbeweglichkeit des Hohlkörpers relativ zu der Aussparung in Steckrichtung zu unterstützen. Die zueinander komplementären Längsprofilierungen ermöglichen eine Verdrehsicherung des Hohlkörpers relativ zur Aussparung und damit relativ zur Verschlussmembran. Zudem bilden sie Gleitführungsprofilierungen, die bei einer Längsverschiebung des Hohlkörpers relativ zur Aussparung Gleitreibungskräfte reduzieren. In Verbindung mit der konischen Gestaltung des Hohlkörpers ergeben sich die gewünschten unterschiedlichen Reibkräfte in entgegengesetzten Richtungen der Steckachse.

In weiterer Ausgestaltung der Erfindung ist der Hohlkörper auf einer zu einem Inneren des Trägergehäuses gewandten Stirnseite durch eine Stützmembran axial gestützt, um ein Lösen des Hohlkörpers aus der Aussparung der Verschlussmembran zu vermeiden. Die Stützmembran gewährleistet, dass der Hohlkörper sich nicht unbeabsichtigt von der Verschlussmembran löst und in dem Trägergehäuse sowie in einem entsprechenden Fluid eines angeschlossenen Fluidspeicherbehältnisses verloren geht.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch eine Verschlussanordnung in einem Längsschnitt,
- Fig. 2: in einem Längsschnitt eine weitere Ausführungsform einer Verschlussanordnung ähnlich Fig. 1,
- Fig. 3: ebenfalls in einem Längsschnitt eine Ausführungsform einer erfindungsgemäßen Verschlussanordnung mit einem separaten Hohlkörper,
- Fig. 4: eine weitere Ausführungsform einer erfindungsgemäßen Verschlussanordnung in einer Längsschnittdarstellung und
- Fig. 5: eine weitere Ausführungsform einer erfindungsgemäßen Verschlussanordnung ähnlich Fig. 4.

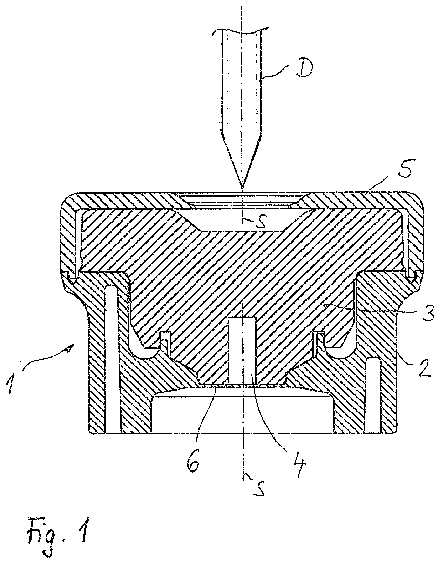

Eine Verschlussanordnung 1, 1a, 1b, 1c, 1d gemäß den Fig. 1 bis 5 ist an einem Trägergehäuse 2, 2a, 2b, 2c, 2d eines medizinischen Fluidspeicherbehältnisses in Form einer Flasche oder eines Beutels vorgesehen, die Teil eines medizinischen Infusions- oder Transfusionssystems sind. Aus Übersichtlichkeitsgründen ist bei allen Ausführungsformen lediglich ein Aufnahmestutzen des Trägergehäuses 2 bis 2d dargestellt, der Teil der jeweiligen Verschlussanordnung 1 bis 1d ist. Der dargestellte Teil des Trägergehäuses 2 bis 2d setzt sich demzufolge fort in ein Gehäuse des Fluidspeicherbehältnisses. Im betriebsfertig montierten Zustand des Infusionssystems ist das entsprechende Fluidspeicherbehältnis derart angeordnet, dass sich der Aufnahmestutzen und demzufolge die Verschlussanordnung 1 bis 1d unten befindet. Vorzugsweise ist das entsprechende Fluidspeicherbehältnis hängend an einem Gestell des Infusions- oder Transfusionssystems gehalten. Zur Verbindung des Fluidspeicherbehältnisses mit entsprechenden Komponenten eines Fluidleitungssystems des medizinischen Infusions- oder Transfusionssystems wird ein Dorn D eines Anschlussteils längs einer Steckachse S in die Verschlussanordnung 1 bis 1d eingestochen. Der Dorn D weist wenigstens einen Kanal zur Fluidleitung auf. Ergänzend kann der Dorn D mit einem Belüftungskanal versehen sein, um ein verbessertes Abfließen des Fluids aus dem Fluidspeicherbehältnis in das Fluidleitungssystem zu ermöglichen.

Bei allen Ausführungsformen gemäß den Fig. 1 bis 5 ist das Trägergehäuse 2 bis 2d im Bereich des Aufnahmestutzens der Verschlussanordnung 1 bis 1d formstabil ausgeführt. Bei allen Ausführungsformen ist in dem Aufnahmestutzen des Trägergehäuses 2 bis 2d eine elastisch deformierbare, stopfenartig gestaltete Verschlussmembran 3 bis 3d vorgesehen, die aus einem Elastomer, vorliegend aus Silikon, hergestellt ist. Die Verschlussmembran 3 bis 3d ist bei allen Ausführungsformen durch eine Abschlusskappe 5 bis 5d formschlüssig auf einem oberen Stirnrand des Aufnahmestutzens des Trägergehäuses 2 bis 2d gesichert. Die Abschlusskappe 5 bis 5d ist zumindest weitgehend formstabil ausgeführt und weist einen zentralen Durchtritt auf, durch den hindurch für den jeweiligen Dorn D der Zugang zum Einstechen in die Verschlussmembran 3 bis 3d gewährleistet ist. Die Verschlussmembran 3 bis 3d schließt den Aufnahmestutzen und damit die entsprechende Öffnung des Trägergehäuses 2a dicht gegen die Umgebung ab.

Um Fluid aus dem Trägergehäuse 2 bis 2d entnehmen zu können, drückt das medizinische Pflegepersonal den Dorn D längs der Steckachse S von außen her durch die Verschlussmembran 3 bis 3d hindurch, bis eine Spitze des Dorns D eine dünne Stützmembran 6 bis 6d durchstochen und demzufolge das Innere des Trägergehäuses erreicht hat. Nun ist ein Durchfluss von Fluid aus dem Fluidspeicherbehältnis durch den Dorn D hindurch zum entsprechenden Fluidleitungssystem ermöglicht. Als Fluid kann eine Infusionslösung oder auch Blut aus einer Blutkonserve vorgesehen sein.

Um bei allen Ausführungsformen gemäß den Fig. 1 bis 5 sicherzustellen, dass der Dorn D, nachdem er einmal die jeweilige Verschlussmembran 3 bis 3d und die Stützmembran 6 bis 6d durchstochen hat, nicht mehr herausgezogen werden kann, sind bei den Verschlussanordnungen 1 bis 1d gemäß den Fig. 1 bis 5 mechanische Rückhaltemittel vorgesehen, die nachfolgend näher beschrieben werden.

Bei der Verschlussanordnung 1 gemäß Fig. 1 ist als mechanisches Rückhaltemittel für den Dorn D eine zylindrische Aussparung 4 in der Verschlussmembran 3 vorgesehen, die zur Stützmembran 6 hin offen ist und sich als Sackloch koaxial zu der Steckachse S von der Innenseite der Verschlussmembran 3 ausgehend in Richtung zur Außenseite der Verschlussmembran 3 zur Abschlusskappe 5 hin erstreckt. Die Länge der Aussparung 4 - längs der Steckachse S gesehen - ist etwas geringer als eine Hälfte einer Dickenerstreckung der Verschlussmembran 3, ebenfalls längs der Steckachse S gesehen. Die Länge der Aussparung 4 ist demzufolge wesentlich geringer als die Dicke der Verschlussmembran 3. Die Aussparung 4 weist einen zylindrischen freien Querschnitt auf, dessen Durchmesser geringer ist als ein Außendurchmesser des Dorns D. Beim Hindurchstechen des Dorns D werden daher die Wandungen der Aussparung 4 zwangsläufig geweitet und elastisch deformiert, so dass die Wandungen der Aussparung 4 sich mit hohem Kraftschluss um den Umfang des Dorns D schmiegen. Durch diesen erhöhten Kraftschluss ist ein Herausziehen des Dorns D in eingestochenem Zustand nur sehr schwer möglich, wodurch der Dorn D mit großer Rückhaltesicherheit in der Verschlussmembran 3 gehalten ist.

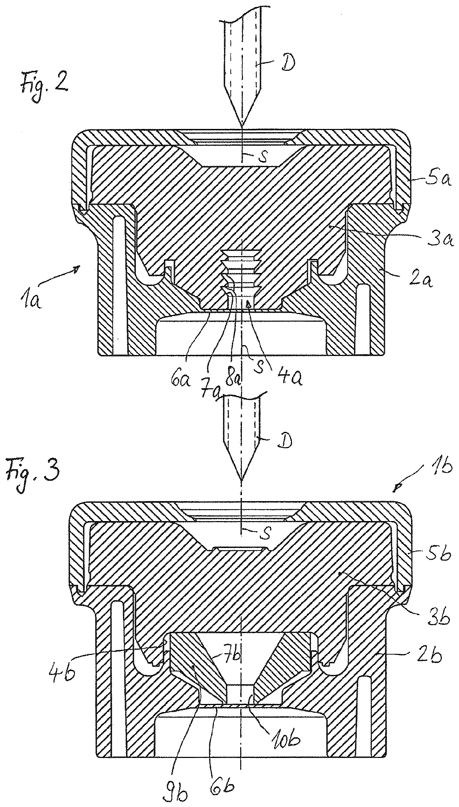

Die Verschlussanordnung 1a gemäß Fig. 2 entspricht im Wesentlichen der zuvor beschriebenen Verschlussanordnung 1 gemäß Fig. 1. Funktionsgleiche Teile und Abschnitte werden mit gleichen Bezugszeichen unter Hinzufügung des Buchstabens a versehen. Einziger Unterschied ist es, dass die Aussparung 4a im Bereich der Innenseite der Verschlussmembran 3a zusätzlich im Bereich ihrer Wandungen mit umlaufenden Rückhalteprofilierungen 7a, 8a versehen ist, die - im Querschnitt gesehen - sägezahnartig profiliert sind. Die Rückhalteprofilierungen sind als zu der Steckachse S koaxiale Ringnuten ausgeführt, die unterschiedliche Profilierungsflanken 7a, 8a bilden. Dabei sind die Profilierungsflanken 7a relativ zur Steckachse S steiler ausgeführt, so dass der Dorn D an den Profilierungsflanken 7a mit reduziertem Reibschluss entlanggleiten kann, sobald er in die Verschlussmembran 3a eingesteckt wird. Die benachbarten Profilierungsflanken 8a jeder Ringnut hingegen sind rechtwinklig zur Steckachse S ausgerichtet, so dass die Profilierungsflanken 8a der Rückhalteprofilierungen bei einem Herausziehen des Dorns D eine größere Rückhaltekraft auf den Außenumfang des Dorns D ausüben als die Profilierungsflanken 7a beim Einstechen des Dorns D. Auch bei der Aussparung 4a ist der freie Durchmesser, der sich über die gesamte Länge der Aussparung 4a erstreckt, kleiner als der Außendurchmesser des Dorns D.

Bei der erfindungsgemäßen Ausführungsform nach Fig. 3 weist die Verschlussanordnung 1b eine Verschlussmembran 3b auf, die im Bereich ihrer Innenseite ebenfalls mit einer Aussparung 4b versehen ist. Diese ist jedoch wesentlich breiter gestaltet als die zuvor beschriebenen Aussparungen 4 und 4a und dient lediglich zur Aufnahme eines separaten Rückhalteelements in Form eines Hohlkörpers 9b. Der Hohlkörper 9b ist trichterartig aus einem Elastomermaterial hergestellt und stützt sich im Bereich seiner zur Abschlusskappe 5b gewandten Stirnseite an einem Boden der Aussparung 4b und im Bereich seiner gegenüberliegenden Stirnseite an der dünnen Stützmembran 6b ab. Zudem verjüngt sich ein Stützbereich des Trägergehäuses 2b von der Verschlussmembran 3b ausgehend zur Stützmembran 6a, die einstückig mit dem Trägergehäuse 2b ausgeführt ist. Dadurch ergibt sich ein zusätzlicher, formschlüssiger Sitz für den Hohlkörper 9b im Trägergehäuse 2b. Der trichterförmige Hohlkörper 9b weist eine sich zur Innenseite, d.h. zur Stützmembran 6b hin konisch verjüngende Innenwandung auf, die eine Profilierungsflanke 7b bildet. Die Profilierungsflanke 7b mündet in einen zylindrischen Durchtrittsabschnitt 10b des Hohlkörpers 9b, dessen Durchmesser geringer ist als der Außendurchmesser des Dorns D.

Anhand der Querschnittsdarstellung der Fig. 3 ist erkennbar, dass der Hohlkörper 9b im Querschnitt allseitig eine Keilform definiert, die zur Innenseite des Trägergehäuses 2b hin geneigt ist. Dadurch ist zwangsläufig ein einfaches Hindurchstecken des Dorns D beim Einstechen in die Verschlussmembran 3b ermöglicht. Bei einem Versuch eines Herausziehens des Dorns D aus dem eingesteckten Zustand hingegen wirkt der Hohlkörper 9b aufgrund seiner elastischen Deformation im Bereich des Durchtrittsabschnitts 10b als zusätzliche, kraftschlüssige Rückhaltesicherung auf den Dorn D.

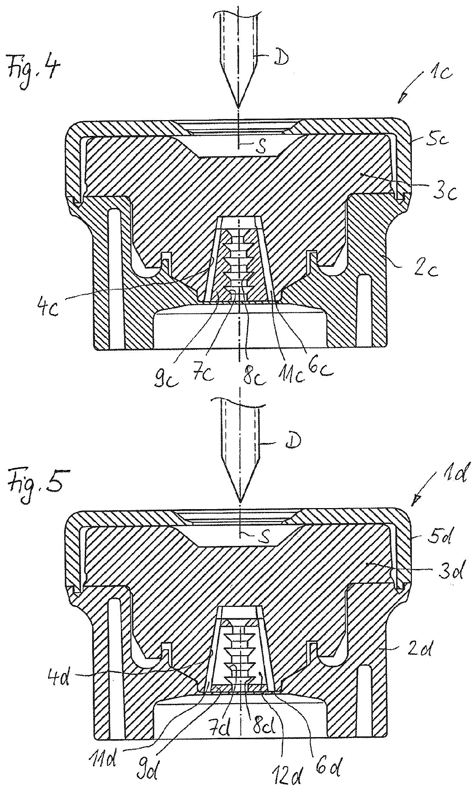

Bei den erfindungsgemäßen Ausführungsformen nach den Fig. 4 und 5 weisen die Verschlussanordnungen 1c und 1d im Bereich der Innenseite der Verschlussmembran 3c, 3d jeweils eine sich zur Außenseite hin koaxial zur Steckachse S konisch verjüngende Aussparung 4c, 4d auf, die ebenfalls als Sackloch ausgeführt ist und sich lediglich über einen Teil der Dicke der Verschlussmembran 3c, 3d erstreckt. Die Innenwandungen der Aussparung 4c, 4d sind mit längs der Steckachse S erstrecken Längsnuten 11c, 11d versehen, die auch als Längsprofilierungen bezeichnet werden. Dabei sind mehrere Längsnuten 11c, 11d über den Umfang der Innenwandung der Aussparung 4c, 4d verteilt angeordnet. In der jeweiligen Aussparung 4c, 4d ist ein im Wesentlichen konischer Hohlkörper 9c, 9d aufgenommen, der mit einem zentralen Durchtrittskanal versehen ist, dessen Durchmesser geringer ist als ein Außendurchmesser des Dorns D. Der jeweilige Hohlkörper 9c, 9d ist im Bereich seines Außenmantels mit komplementären Längsprofilierungen versehen, die in die Längsnuten 11c, 11d eingreifen, um eine Verdrehsicherung des Hohlkörpers 9c, 9d in der entsprechenden Aussparung 4c, 4d zu gewährleisten und gleichzeitig eine Längsverschiebbarkeit des jeweiligen Hohlkörpers 9c, 9d längs der Steckachse S innerhalb der Aussparung 4c, 4d zu vereinfachen. Bei beiden Hohlkörpern 9c, 9d ist der Durchtrittskanal zusätzlich mit ringnutartigen Rückhalteprofilierungen 7c, 8c; 7d, 8d versehen, die unterschiedlich geneigte Profilierungsflanken bilden analog der Profilierungsflanken der Aussparung 4a der Ausführungsform nach Fig. 2. Zur Vermeidung von Wiederholungen wird daher auf die Zeichnungen gemäß den Fig. 4 und 5 sowie auf die Beschreibung zur Fig. 2 verwiesen.

Bei der Ausführungsform nach Fig. 4 ist der Hohlkörper 9c als über seine Mantelfläche durchgängig geschlossener Hohlkörper ausgeführt. Bei der Ausführungsform nach Fig. 5 hingegen weist der Hohlkörper 9d im Bereich seiner Mantelfläche mehrere fensterartige Längsschlitze 12d auf, die über den Umfang des Hohlkörpers 9d verteilt angeordnet sind. Die Längsschlitze sind sowohl seitlich nach außen als auch seitlich nach innen zum Durchtrittskanal hin offen und gewährleisten eine einfachere elastische Deformierbarkeit des Hohlkörpers 9d gegenüber dem geschlossenen Hohlkörper 9c nach Fig. 4. Wie anhand der Fig. 5 erkennbar ist, erstrecken sich die Längsschlitze 12d lediglich über einen Teilbereich der Länge des Hohlkörpers 9d - auf die Steckachse S bezogen.

## Patentansprüche

1. Verschlussanordnung (1 bis 1d) für ein Trägergehäuse (2 bis 2d) eines medizinischen Fluidspeicher- und/oder -leitungssystems mit einem Aufnahmestutzen, in dem eine elastisch verformbare Verschlussmembran (3 bis 3d) angeordnet ist, die so gestaltet ist, dass sie einen eingesteckten Dorn (D) eines Anschlussteils kraftschlüssig sichert, wobei die Verschlussmembran (3 bis 3d) wenigstens ein mechanisches Rückhaltemittel (4, 4a; 9b bis 9d) aufweist, das auf den Dorn (D) des Anschlussteils in eingestecktem Zustand derart wirkt, dass höhere Kräfte zum Herausziehen des Dorns (D) aus der Verschlussmembran (3 bis 3d) notwendig sind als die Kräfte, die für ein Hineinstecken des Dorns (D) benötigt sind, und wobei das wenigstens eine Rückhaltemittel ein separates Rückhalteelement aus Kunststoff umfasst, das lösbar oder unlösbar mit der Verschlussmembran (3b bis 3d) verbunden ist, **dadurch gekennzeichnet, dass** das Rückhalteelement als elastisch verformbarer Hohlkörper (9b, 9c, 9d) gestaltet ist, der an seinem Innenumfang Rückhalteprofilierungen (7b, 8b; 7c, 8c; 7d, 8d) aufweist, deren Profilflanken in Einsteckrichtung steiler gestaltet sind als in Entnahmerichtung.

2. Verschlussanordnung nach Anspruch 1, **dadurch gekennzeichnet, dass** der Hohlkörper (9c, 9d) konisch gestaltet ist, und dass die Verschlussmembran (3c, 3d) eine komplementär konische Aussparung (4c, 4d) aufweist, in die der Hohlkörper (9c, 9d) eintaucht.

3. Verschlussanordnung nach Anspruch 2, **dadurch gekennzeichnet, dass** die konische Form des Hohlkörpers (9c, 9d) und der Aussparung (4c, 4d) sich von der Innenseite der Verschlussmembran (3c, 3d) in Richtung zur Außenseite verjüngt.

4. Verschlussanordnung nach einem der Ansprüche 1 bis 3, **dadurch gekennzeichnet, dass** der Hohlkörper (9b) mit über seinen Umfang verteilt angeordneten seitlichen Längsschlitzen (12d) versehen ist, die eine elastische Deformation unterstützen.

5. Verschlussanordnung nach einem der Ansprüche 1 bis 4, **dadurch gekennzeichnet, dass** die Aussparung (4c, 4d) zur Aufnahme des Hohlkörpers (9c, 9d) mit Längsprofilierungen (11c, 11d) versehen ist.

6. Verschlussanordnung nach Anspruch 5, **dadurch gekennzeichnet, dass** ein Außenmantel des Hohlkörpers (9c, 9d) mit komplementären Längsprofilierungen versehen ist, um eine Gleitbeweglichkeit des Hohlkörpers (9c, 9d) relativ zu der Aussparung (4c, 4d) längs der Steckachse (S) zu unterstützen.

7. Verschlussanordnung nach einem der Ansprüche 1 bis 6, **dadurch gekennzeichnet, dass** der Hohlkörper (9b bis 9d) auf einer zu einem Inneren des Trägergehäuses (2b bis 2d) gewandten Stirnseite durch eine Stützmembran (6b bis 6d) axial gestützt ist, um ein Lösen des Hohlkörpers (9b bis 9d) aus der Aussparung (4b bis 4d) der Verschlussmembran (3b bis 3d) zu vermeiden.

## Claims

1. Closure assembly (1 to 1d) for a carrier housing (2 to 2d) of a medical fluid storage and/or conduit system, comprising an intake connection piece, wherein an elastically deformable closure diaphragm (3 to 3d) is disposed and configured such that an inserted pin (D) of a connector part is secured in a force-fitting manner, wherein the closure diaphragm (3 to 3d) has at least one mechanical retaining means (4, 4a; 9b to 9d) acting on the pin (D) of the connector part in the inserted condition such that forces necessary for extracting the pin (D) from the closure diaphragm (3 to 3d) are superior to forces needed for inserting the pin (D), and wherein the at least one retaining means comprises a separate retaining element made of synthetic material, which is detachably or non-detachably connected to the closure diaphragm (3b to 3d),
**characterized in that**
the retaining element is designed as an elastically deformable hollow body (9b, 9c, 9d) including retaining profiles (7b, 8b; 7c, 8c; 7d, 8d) on the interior circumference thereof, with the profile edges having a steeper slope in the inserting direction than in the extracting direction.

2. Closure assembly according to claim 1, **characterized in that** the hollow body (9c, 9d) has a conical design, and **in that** the closure diaphragm (3c, 3d) has a complementary conical recess (4c, 4d) for the hollow body (9c, 9d) to plunge in.

3. Closure assembly according to claim 2, **characterized in that** the conical shapes of the hollow body (9c, 9d) and the recess (4c, 4d) taper from the interior side of the closure diaphragm (3c, 3d) towards the exterior side.

4. Closure assembly according to any of claims 1 to 3, **characterized in that** the hollow body (9b) is provided with lateral longitudinal slots (12d) disposed distributed over the circumference thereof to support elastic deformation.

5. Closure assembly according to any of claims 1 to 4, **characterized in that** the recess (4c, 4d) is provided with longitudinal profiles (11c, 11d) for accommodating the hollow body (9c, 9d).

6. Closure assembly according to claim 5, **characterized in that** an outer shell of the hollow body (9c, 9d) is provided with complementary longitudinal profiles, in order to support sliding mobility of the hollow body (9c, 9d) in relation to the recess (4c, 4d) along the insertion axis (S).

7. Closure assembly according to any of claims 1 to 6, **characterized in that** the hollow body (9b to 9d) is axially supported by a support diaphragm (6b to 6d) on a face side oriented towards an interior of the carrier housing (2b to 2d), in order to prevent detachment of the hollow body (9b to 9d) from the recess (4b to 4d) of the closure diaphragm (3b to 3d).

## Revendications

1. Ensemble de fermeture (1 à 1d) pour un boîtier de support (2 à 2d) d'un système médical de récipients et/ou de conduits à fluide, présentant une tubulure de réception dans laquelle est disposée une membrane de fermeture (3 à 3d) élastiquement déformable configurée de telle sorte qu'elle bloque en correspondance mécanique un mandrin (D) d'une pièce de raccordement qui y a été enfichée,
la membrane de fermeture (3 à 3d) présentant au moins un moyen mécanique de retenue (4, 4a; 9b à 9d) qui agit sur le mandrin (D) de la pièce de raccordement lorsqu'elle a été enfichée de telle sorte que la force nécessaire pour extraire le mandrin (D) hors de la membrane de fermeture (3 à 3d) soit supérieure à la force nécessaire pour enficher le mandrin (D),
le ou les moyens de retenue comprenant un élément de retenue séparé en matière synthétique relié de manière libérable ou non libérable à la membrane de fermeture (3b à 3d),
**caractérisé en ce que**
l'élément de retenue est maintenu en tant que corps creux élastiquement déformable (9b, 9c, 9d) qui présente à sa périphérie intérieure un profil de retenue (7b, 8b; 7c, 8c; 7d, 8d) dont les flancs sont plus accusés dans la direction d'insertion que dans la direction de prélèvement.

2. Ensemble de fermeture selon la revendication 1, **caractérisé en ce que** le corps creux (9c, 9d) est de forme conique et **en ce que** la membrane de fermeture (3c, 3d) présente une découpe conique (4c, 4d) complémentaire dans laquelle s'enfonce le corps creux (9c, 9d).

3. Ensemble de fermeture selon la revendication 2, **caractérisé en ce que** la forme conique du corps creux (9c, 9d) et de la découpe (4c, 4d) se rétrécit du côté intérieur de la membrane de fermeture (3c, 3d) en direction de son côté extérieur.

4. Ensemble de fermeture selon l'une des revendications 1 à 3, **caractérisé en ce que** le corps creux (9b) est doté de fentes longitudinales latérales (12d) réparties à sa périphérie et soutenant une déformation élastique.

5. Ensemble de fermeture selon l'une des revendications 1 à 4, **caractérisé en ce que** la découpe (4c, 4d) est dotée de profils longitudinaux (11c, 11d) en vue de reprendre le corps creux (9c, 9d).

6. Ensemble de fermeture selon la revendication 5, **caractérisé en ce qu'**une enveloppe extérieure du corps creux (9c, 9d) est dotée de profilés longitudinaux complémentaires qui soutiennent la possibilité d'un déplacement par coulissement du corps creux (9c, 9d) par rapport à la découpe (4c, 4d) dans la direction de l'axe d'enfichage (S).

7. Ensemble de fermeture selon l'une des revendications 1 à 6, **caractérisé en ce que** le corps creux (9b à 9d) est soutenu axialement par une membrane de soutien (6b à 6d) sur un côté frontal tourné vers l'intérieur du boîtier de support (2b à 2d), pour empêcher un détachement du corps creux (9b à 9d) hors de la découpe (4b à 4d) de la membrane de fermeture (3b à 3d).
